# EUROPÄISCHE PATENTSCHRIFT

(11) **EP 0 642 776 B1**
(45) Veröffentlichungstag und Bekanntmachung des Hinweises auf die Patenterteilung: **14.04.1999**
(21) Anmeldenummer: 94810486.4
(22) Anmeldetag: 25.08.1994
(51) Int. Cl.: A61F 9/02

(54) **Verfahren zur Steuerung einer Blendschutzeinrichtung und Blendschutzeinrichtung zur Durchführung des Verfahrens**
Method to control an anti-glare device and anti-glare device used in the method
Méthode et contrôle d'un dispositif anti-éblouissant et dispositif anti-éblouissant selon la méthode

(30) Priorität: 13.09.1993 DE 4330817
(43) Veröffentlichungstag der Anmeldung: 15.03.1995
(73) Patentinhaber: OPTREL AG, 9630 Wattwil (CH)
(72) Erfinder: Ackermann, Emil, CH-9630 Wattwil (CH)
(74) Vertreter: Rottmann, Maximilian R.

(56) Entgegenhaltungen:
- DE-A- 3 437 704
- DE-A- 4 106 019
- DE-A- 4 118 208
- GB-A- 2 137 373
- US-A- 4 462 661
- US-A- 4 848 890
- US-A- 5 059 814

## Beschreibung

Die vorliegende Erfindung betrifft ein Verfahren zur Steuerung einer Blendschutzeinrichtung, welche mit einer bezüglich ihrer optischen Transmission elektrisch steuerbaren Filterscheibe und einer mit dieser in Wirkverbindung stehenden, einen lichtempfindlichen Sensor umfassenden elektronischen Steuereinheit ausgerüstet ist, mit dem Zweck, das Mass der optischen Transmission der Filterscheibe gegensinnig zur Intensität des auf den Sensor fallenden Lichts zu verändern.

Blendschutzeinrichtungen dieser Art sind in zahlreichen Ausführungs-und Anwendungsformen bekannt geworden und beispielsweise in den Patentschriften EP 0 091 514 B1 und DE 41 28 291 C2 beschrieben. Bisher fanden solche Blendschutzeinrichtungen vorzugsweise Anwendung an Schutzbrillen, Schutzhelmen und Schutzschildern in der Schweiss- und Schneidbrennertechnik. Im Hinblick auf die zunehmende Miniaturisierung elektronischer Schaltungsanordnungen besteht aber vermehrt auch die Möglichkeit, derartige Blendschutzeinrichtungen z.B. an Sonnenbrillen und an Schutzhelmen für Zweiradfahrer vorzusehen.

Als Filterscheibe mit regelbarer optischer Transmission dient vorzugsweise eine Flüssigkristallzelle. In der Regel bildet diese einen Teil einer Filteranordnung, welche noch andere Filterelemente umfasst, z.B. solche, welche den Durchlassbereich der Filteranordnung im wesentlichen auf das sichtbare Licht beschränken. Für die Erfassung der Intensität des auf die Filterscheibe fallenden Lichts ist normalerweise ein lichtempfindlicher Sensor in Form eines optoelektrischen Wandlers vorgesehen, der sowohl im Strahlengang der Filteranordnung als auch unmittelbar neben der Filteranordnung angeordnet sein kann. Es sind auch Ausführungen bekannt, die mehr als einen derartigen Wandler aufweisen.

Für den im Auge hervorgerufenen Helligkeitseindruck ist im wesentlichen die Beleuchtungsstärke massgebend. Mit dem lichtempfindlichen Sensor soll das Mass der am Auge zu erwartenden Beleuchtungsstärke ermittelt werden, um damit über die elektronische Steuereinheit den Grad der optischen Transmission der steuerbaren Filterscheibe einzustellen. Dabei kann die elektronische Steuereinheit so ausgelegt sein, dass deren Wirkung erst dann einsetzt, wenn bei zunehmender Lichtstärke oder Leuchtdichte die Beleuchtungsstärke einen bestimmten Grenzwert überschreitet. Dieser Grenzwert kann so gewählt werden, dass eine Verminderung der optischen Transmission nur dann eingeleitet wird, wenn eine weitere Erhöhung der gemessenen Leuchtdichte zu einer Blendwirkung führen könnte. Damit soll vermieden werden, dass das Blickfeld bei gleichmässig starker, aber blendfreier Beleuchtung unnötigerweise verdunkelt wird.

Das von einem optoelektrischen Wandler, z.B. einer Photodiode oder dergleichen, aus verschiedenen Richtungen aufgenommene Licht fällt im allgemeinen mit unterschiedlicher Lichtstärke auf die exponierte Sensorfläche. Insbesondere beim Vorhandensein punktförmiger Lichtquellen mit intensiven Lichtstärken und hohen Leuchtdichten, z.B. Schweisslichtbögen, können die Lichtstärken in dem vom Sensor überwachten Raum örtlich stark variieren. Der optoelektrische Wandler kann aber diese örtlichen Lichtstärkeunterschiede nicht erfassen; er erzeugt vielmehr ein Ausgangssignal, das einem Mittelwert der Beleuchtungsstärke entspricht.

Dieser Umstand ist insbesondere dann von Nachteil, wenn die Lichtstärken in dem vom Sensor überwachten Raum örtlich stark variieren. Beim Vorhandensein einer punktförmigen Lichtquelle mit extrem hoher Lichtstärke in lichtschwachem Umfeld können wegen dieser Mittelwertbildung Ausgangssignale entstehen, deren Wert nicht wesentlich grösser ist als derjenige eines Ausgangssignals, das sich aufgrund einer verhältnismässig starken Raumhelligkeit ergibt. Dabei kann aber eine punktförmige Lichtquelle eine weit höhere Blendwirkung bewirken als andere, flächenmässig ausgedehntere Lichtquellen. Unter diesen Umständen fällt es schwer, mit einem solchen Sensor die verschiedenartigen Lichtquellen voneinander zu unterscheiden und deren Blendwirkung abzuschätzen. Dies kann bei einer Steuereinheit, welche auf einen bestimmten Grenzwert der Beleuchtungsstärke anspricht, sogar dazu führen, dass einerseits beim Vorhandensein einer punktförmigen Lichtquelle mit hoher Blendwirkung ein Abdunkeln der Filterscheibe unterbleibt, während andererseits bei starker, aber blendfreier Raumbeleuchtung oder allein durch allenfalls vorhandene Störlichtquellen die Filterscheibe unerwünschterweise dunkel gesteuert wird.

Ein Verfahren und eine Vorrichtung gemäß Oberbegriff der Ansprüchen 1 und 2 ist aus der DE-A-41 18 208. Gemäß DE-A-41 18 208 wird polarisiertes Licht durch elektrisch angesteuerte Flüssigkristallelemente hindurch gestrahlt. Über optische Sensoren werden eine oder mehrere charakteristische Eigenschaften des Lichts, z.B. die Intensität in Abhängigkeit vom Einfallswinkel, detektiert. Die so erhaltenen Signale werden zur Ansteuerung der Flüssigkristallelemente verwendet. Mit dieser Vorrichtung lässt sich das zuvor umrissene Problem nicht lösen, da die Richtung, aus der eine als störend empfundene, starke, punktförmige Lichtquelle strahlt, üblicherweise nicht bekannt ist bzw. variieren kann.

Aus der US-A-4 848 890 ist eine Sonnenbrille bekannt, die den Benutzer vor direktem Sonnenlicht schützen soll. Zu diesem Zweck sind matrixartige LCD-Elemente vorgesehen, bei denen einzelne Bereiche, je nach einfallendem Sonnenlicht, gezielt abgedunkelt werden können, währenddem die restlichen Bereiche unverändert bleiben. Der Aufwand für eine Ausbildung mit matrixartigen LCD-Elementen und insbesondere für die individuelle Steuerung deren einzelner Bereiche ist aber unverhältnismässig hoch.

In der Patentschrift DE 34 37 704 A1 ist eine Blendschutzeinrichtung beschrieben, bei welcher dem Sensor eine abbildende Optik vorgeschaltet ist, welche die Lichtquellen in dem vom Sensor überwachten Raum auf die exponierte Fläche des Sensors abbildet, und bei welcher sowohl der Sensor als auch die Filterscheibe rasterförmig in mehrere, getrennt arbeitende Elemente aufgeteilt sind, wobei jedes Sensorelement über eine Steuereinheit ein ihm flächenmässig zugeordnetes Filterscheibenelement ansteuert. Auf diese Weise werden z.B. diejenigen Elemente der Filterscheibe am stärksten abgedunkelt, welche von der höchsten Lichtdichte betroffen sind, während andere Elemente der Filterscheibe weniger oder unter Umständen gar nicht abgedunkelt werden. Mit einer derartigen, eine Vielzahl von Sensorelementen aufweisenden Sensormatrix lassen sich zwar punktförmige, starke Lichtquellen und flächenmässig ausgedehnte Lichtquellen in Bezug auf ihre Blendwirkung besser voneinander unterscheiden; der Aufwand für eine entsprechend hohe Anzahl von Filterscheibenelementen und für die Mittel zur individuellen Steuerung derselben ist jedoch verhältnismässig hoch. Ausserdem wird durch die örtlich unterschiedliche Abdunklung des Blickfeldes der Bildkontrast unter Umständen in unnatürlicher bzw. störender Weise verändert.

Die vorliegende Erfindung hat zum Ziel, ein Verfahren zur Steuerung einer Blendschutzeinrichtung der eingangs genannten Art anzugeben, das unter Vermeidung der vorerwähnten Nachteile ein eindeutiges Kriterium für die Erfassung der eine Blendwirkung hervorrufenden Beleuchtungsstärke liefert und mit einer ganzflächigen Filterscheibe und mit einem einzigen Signal für die Steuerung der optischen Transmission derselben auskommt.

Diese Aufgabe wird erfindungsgemäss dadurch gelöst, dass die vom Sensor erfassten Lichtquellen auf der exponierten Fläche des Sensors abgebildet werden und dass die Intensität des einfallenden Lichts mit einer Vielzahl von Flächenelementen der Sensorfläche selektiv gemessen und das jeweils höchste Messignal ausgewählt und zur einheitlichen Steuerung der optischen Transmission der ganzen Filterscheibe ausgewertet wird.

Auf diese Weise gelingt es nicht nur, eine punktförmige, Blendung verursachende Lichtquelle eindeutig als solche zu erfassen; vielmehr lässt sich die Steuerung der optischen Transmission durch deren einheitliche Anwendung auf die gesamte Filterscheibe auch wesentlich vereinfachen. Ausserdem wird in diesem Fall durch die Änderung der optischen Transmission der Bildkontrast nicht verfälscht.

Die Erfindung betrifft auch eine Blendschutzeinrichtung gemäss dem Oberbegriff des Patentanspruchs 2. Sie ist dadurch gekennzeichnet, dass die elektronische Steuereinheit eine Schaltungsanordnung zur Auswahl des jeweils höchsten der an den Wandlerelementen anfallenden Ausgangssignale für die einheitliche Steuerung der optischen Transmission der ganzen Filterscheibe aufweist.

Vorzugsweise ist dem lichtempfindlichen Sensor eine abbildende Optik vorgeschaltet, welche die Lichtquellen in dem vom Sensor überwachten Raum auf die exponierte Fläche des Sensors abbildet. Unter Umständen kann es zweckmässig sein, dem lichtempfindlichen Sensor auch ein Filter für optische Strahlung vorzuschalten.

Anhand der beigefügten Zeichnung wird das erfindungsgemässe Verfahren an einem Ausführungsbeispiel der Blendschutzeinrichtung näher erläutert. In der Zeichnung bedeuten:
- Fig. 1: eine Frontansicht einer erfindungsgemässen Blendschutzeinrichtung in schematischer Darstellung;
- Fig. 2: einen Querschnitt durch die Blendschutzeinrichtung nach der Linie A-A in Fig. 1;
- Fig. 3: eine Ansicht eines lichtempfindlichen Sensors in grösserem Massstab; und
- Fig. 4: eine vergrösserte Darstellung eines einzelnen Sensorelements.

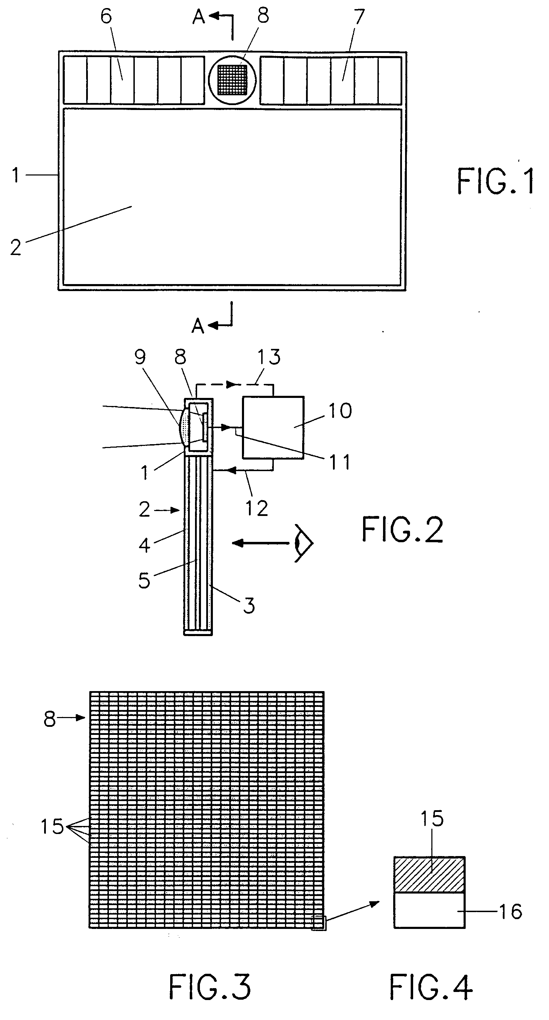

Die in den Fig. 1 und 2 dargestellte Blendschutzeinrichtung enthält in einem Gehäuserahmen 1 ein Sichtfenster 2, in das eine bezüglich ihrer optischen Transmission elektrisch steuerbare Filterscheibe 3, z.B. in Form einer Flüssigkristallzelle, eingesetzt ist. Der steuerbaren Filterscheibe 3 sind in an sich bekannter Weise ein UV-Filter 4 und ein IR-Filter 5 vorgeschaltet. Oberhalb des Sichtfensters 2 sind Solarzellen 6 und 7 für die Stromversorgung der Blendschutzeinrichtung vorgesehen. Dazwischen befindet sich ein lichtempfindlicher Sensor 8 mit vorgeschalteter Optik 9. Der Sensor 8 ist einer elektronischen Steuereinheit 10 zugeordnet, welche vom Sensor 8 über die Signalverbindung 11 beeinflusst wird und über die Signalverbindung 12 die optische Transmission der Filterscheibe 3 steuert. Über die Leitung 13 erfolgt die Stromversorgung der Steuereinheit 10 aus den Solarzellen 6 und 7.

Der lichtempfindliche Sensor 8 weist gemäss den Fig. 3 und 4 eine Vielzahl von in einem Flächenraster angeordneten optoelektrischen Wandlerelementen 15 mit getrennt erfassbaren Ausgangssignalen auf. Beispielsweise sind auf einer quadratischen Fläche 25 mal 25, also insgesamt 625 Wandlerelemente 15 vorgesehen. Diese Wandlerelemente 15, vorzugsweise Photodioden, sind auf einer gemeinsamen Halbleiterscheibe ausgebildet, auf welcher ausserdem je ein Platz 16 für den Wandlerelementen 15 einzeln zugeordnete Schaltungselemente der elektronischen Steuereinheit 10 vorhanden ist.

Bei den genannten, zur Steuereinheit 10 gehörenden Schaltungselementen handelt es sich in erster Linie um Teile einer Schaltungsanordnung zur Auswahl des jeweils höchsten der an den Wandlerelementen 15 anfallenden Ausgangssignale. Solche Auswahlschaltungen und deren Wirkungsweise sind an sich bekannt und beispielsweise in der US-Patentschrift 5 059 814 ausführlich beschrieben. Grundsätzlich arbeiten diese Auswahlschaltungen so, dass von den einer gemeinsamen Sammelleitung zugeführten Ausgangssignalen dasjenige mit dem höchsten Signalwert das Potential auf der Sammelleitung bestimmt, während die Wirkung aller übrigen Ausgangssignale mit geringerem Signalwert praktisch vollständig unterdrückt wird.

Selbstverständlich besteht auch die Möglichkeit, weitere elektronische Schaltungselemente der Steuereinheit 10 auf der den lichtempfindlichen Sensor 8 bildenden Halbleiterscheibe anzudrohenden. Mit Ausnahme von manuell zu betätigenden Steuermitteln, kann die gesamte Steuereinheit 10 zusammen mit dem Sensor 8 eine Baueinheit bilden.

## Patentansprüche

1. Verfahren zur Steuerung einer Blendschutzeinrichtung, welche mit einer bezüglich ihrer optischen Transmission elektrisch steuerbaren Filterscheibe (3) und einer mit dieser in Wirkverbindung stehenden, einen lichtempfindlichen Sensor (8) umfassenden elektronischen Steuereinheit (10) ausgerüstet ist, mit dem Zweck, das Mass der optischen Transmission der Filterscheibe (3) gegensinnig zur Intensität des auf den Sensor (8) fallenden Lichts zu verändern, wobei die vom Sensor (8) erfassten Lichtquellen auf der exponierten Fläche des Sensors (8) abgebildet werden und die Intensität des einfallenden Lichts mit einer Vielzahl von Flächenelementen (15) der Sensorfläche selektiv gemessen wird, dadurch gekennzeichnet, dass das jeweils höchste Messignal ausgewählt und zur einheitlichen Steuerung der optischen Transmission der ganzen Filterscheibe (3) ausgewertet wird.

2. Blendschutzeinrichtung zur Durchführung des Verfahrens nach Anspruch 1, mit einer bezüglich ihrer optischen Transmission elektrisch steuerbaren Filterscheibe (3) und einer mit dieser in Wirkverbindung stehenden, einen lichtempfindlichen Sensor (8) umfassenden elektronischen Steuereinheit (10), wobei Mittel zur Abbildung der Lichtquellen in dem vom Sensor überwachten Raum auf die exponierte Fläche des Sensors vorgesehen sind und der lichtempfindliche Sensor (8) eine Vielzahl von in einem Flächenraster angeordneten optoelektrischen Wandlerelementen (15) mit getrennt erfassbaren Ausgangssignalen aufweist, dadurch gekennzeichnet, dass die elektronische Steuereinheit (10) eine Schaltungsanordnung zur Auswahl des jeweils höchsten der an den Wandlerelementen (15) anfallenden Ausgangssignale für die einheitliche Steuerung der optischen Transmission der ganzen Filterscheibe (3) aufweist.

3. Blendschutzeinrichtung nach Anspruch 2, dadurch gekennzeichnet, dass die optoelektrischen Wandlerelemente (15) Photodioden sind.

4. Blendschutzeinrichtung nach einem der Ansprüche 2 und 3, dadurch gekennzeichnet, dass die optoelektrischen Wandlerelemente (15) auf einer gemeinsamen Halbleiterscheibe ausgebildet sind.

5. Blendschutzeinrichtung nach Anspruch 2, dadurch gekennzeichnet, dass wenigstens ein Teil der elektronischen Steuereinheit (10) mit dem lichtempfindlichen Sensor (8) eine Baueinheit bildet.

6. Blendschutzeinrichtung nach den Ansprüchen 4 und 5, dadurch gekennzeichnet, dass die den optoelektrischen Wandlerelementen (15) einzeln zugeordneten Schaltungselemente der Schaltungsanordnung zur Auswahl des höchsten Ausgangssignals zusammen mit den optoelektrischen Wandlerelementen (15) auf einer gemeinsamen Halbleiterscheibe ausgebildet sind.

7. Blendschutzeinrichtung nach einem der Ansprüche 2 bis 6, dadurch gekennzeichnet, dass dem lichtempfindlichen Sensor (8) eine abbildende Optik (9) vorgeschaltet ist, welche die Lichtquellen in dem vom Sensor überwachten Raum auf die exponierte Fläche des Sensors (8) abbildet.

8. Blendschutzeinrichtung nach einem der Ansprüche 2 bis 7, dadurch gekennzeichnet, dass dem lichtempfindlichen Sensor (8) ein Filter für optische Strahlung vorgeschaltet ist.

9. Blendschutzeinrichtung nach einem der Ansprüche 2 bis 8, dadurch gekennzeichnet, dass Solarzellen (6, 7) zur Energieversorgung der Blendschutzeinrichtung vorgesehen sind.

## Claims

1. Method for controlling an anti-glare device which is fitted with a filter disc (3), which can be controlled electrically with reference to its optical transmission, and with an electronic control unit (10), which is operationally connected to said filter disc and comprises a light-sensitive sensor (8), with the aim of varying the dimension of the optical transmission of the filter disc (3) inversely with respect to the intensity of the light failing on the sensor (8), the light sources detected by the sensor (8) being imaged on the exposed surface of the sensor (8), and the intensity of the incident light being selectively measured by means of a multiplicity of surface elements (15) of the sensor surface, characterized in that the currently highest measuring signal is selected and evaluated for the purpose of uniform control of the optical transmission of the entire filter disc (3).

2. Anti-glare device for carrying out the method according to Claim 1, having a filter disc (3), which can be controlled electrically with reference to its optical transmission, and an electronic control unit (10) which is operationally connected to said filter disc and comprises a light-sensitive sensor (8), it being the case that means are provided for imaging the light sources in the space monitored by the sensor on the exposed surface of the sensor, and that the light-sensitive sensor (8) has a multiplicity of optoelectric transducer elements (15) which are arranged in a surface-area grid and have separately detectable output signals, characterized in that the electronic control unit (10) has a circuit arrangement for selecting the currently highest of the output signals arriving at the transducer elements (15) for the uniform control of the optical transmission of the entire filter disc (3).

3. Anti-glare device according to Claim 2, characterized in that the optoelectric transducer elements (15) are photodiodes.

4. Anti-glare device according to one of Claims 2 and 3, characterized in that the optoelectric transducer elements (15) are constructed on a common semiconductor wafer.

5. Anti-glare device according to Claim 2, characterized in chat at least a part of the electronic control unit (10) forms a subassembly with the light-sensitive sensor (8).

6. Anti-glare device according to Claims 4 and 5, characterized in that the circuit elements of the circuit arrangement which are assigned individually to the optoelectric transducer elements (15) are constructed together with the optoelectric transducer elements (15) on a common semiconductor wafer in order to select the highest output signal.

7. Anti-glare device according to one of Claims 2 to 6, characterized in that connected upstream of the light-sensitive sensor (8) is an imaging optical system (9) which images the light sources in the space monitored by the sensor on the exposed surface of the sensor (8).

8. Anti-glare device according to one of Claims 2 to 7, characterized in that a filter for optical radiation is connected upstream of the light-sensitive sensor (8).

9. Anti-glare device according to one of Claims 2 to 8, characterized in that solar cells (6, 7) are provided for supplying power to the anti-glare device.

## Revendications

1. Procédé de commande d'un dispositif antiéblouissement, qui est pourvu d'une plaque-filtre (3), pouvant être commandée électriquement en ce qui concerne sa transmission optique, et d'une unité électronique de commande (10) coopérant avec cette plaque-filtre et comprenant un capteur (8) photosensible, dans le but de faire varier l'amplitude de la transmission optique de la plaque-filtre (3) dans le sens opposé à l'intensité de la lumière incidente sur le capteur (8), selon lequel les sources de lumière détectées par le capteur (8) font l'objet d'une formation d'image sur la surface exposée du capteur (8) et l'intensité de la lumière incidente est mesurée sélectivement au moyen de multiples éléments de surface (15) de la surface de capteur, caractérisé en ce que le signal de mesure chaque fois le plus élevé est sélectionné et exploité pour la commande commune de la transmission optique de l'ensemble de la plaque-filtre (3).

2. Dispositif antiéblouissement pour la mise en oeuvre du procédé de la revendication 1, pourvu d'une plaque-filtre (3), pouvant être commandée électriquement en ce qui concerne sa transmission optique, et d'une unité électronique de commande (10) coopérant avec cette plaque-filtre et comprenant un capteur (8) photosensible, des moyens étant prévus pour la formation d'image des sources de lumière, situées dans l'espace surveillé par le capteur, sur la surface exposée du capteur, et le capteur (8) photosensible comprenant de multiples éléments convertisseurs optoélectriques (15) disposés suivant un réseau de surface et présentant des signaux de sortie pouvant être détectés séparément caractérisé en ce que l'unité électronique de commande (10) comprend un agencement de circuit pour la sélection de celui qui est chaque fois le plus élevé parmi les signaux de sortie se présentant sur les éléments convertisseurs (15), pour la commande commune de la transmission optique de l'ensemble de la plaque-filtre (3).

3. Dispositif antiéblouissement suivant la revendication 2, caractérisé en ce que les éléments convertisseurs opto-électriques (15) sont des photodiodes.

4. Dispositif antiéblouissement suivant l'une des revendications 2 et 3, caractérisé en ce que les éléments convertisseurs opto-électriques (15) sont disposés sur une plaque de semi-conducteur commune.

5. Dispositif antiéblouissement suivant la revendication 2, caractérisé en ce qu'au moins une partie de l'unité électronique de commande (10) forme avec le capteur (8) photosensible une unité structurelle.

6. Dispositif antiéblouissement suivant les revendications 4 et 5, caractérisé en ce que les éléments de circuit de l'agencement de circuit, servant à la sélection du signal de sortie le plus élevé, qui sont associés individuellement aux éléments convertisseurs opto-électriques (15) sont réalisés en commun avec les éléments convertisseurs optoélectriques (15) sur une plaque de semi-conducteur commune.

7. Dispositif antiéblouissement suivant l'une des revendications 2 à 6, caractérisé en ce qu'il est prévu en amont du capteur (8) photosensible une optique de formation d'image (9) qui forme une image des sources de lumière, situées dans l'espace surveillé par le capteur, sur la surface exposée du capteur (8).

8. Dispositif antiéblouissement suivant l'une des revendications 2 à 7, caractérisé en ce qu'un filtre pour un rayonnement optique est disposé en amont du capteur (8) photosensible.

9. Dispositif antiéblouissement suivant l'une des revendications 2 à 8, caractérisé en ce que des cellules polaires (6, 7) sont prévues pour, l'alimentation en énergie du dispositif antiéblouissement.
